# EUROPEAN PATENT SPECIFICATION

(11) **EP 1 677 811 B1**
(45) Date of publication and mention of the grant of the patent: **10.09.2008**
(21) Application number: 04769054.0
(22) Date of filing: 28.07.2004
(51) Int. Cl.: A61K 35/64, A61P 31/00, A61P 35/00

(54) **MEDICAMENT ON THE BASIS OF HONEY, PREPARATION AND USE THEREOF**
MEDIKAMENT AUF BASIS VON HONIG, SEINE HERSTELLUNG UND VERWENDUNG
MEDICAMENT A BASE DE MIEL, SA PREPARATION ET SON UTILISATION

(30) Priority: 17.10.2003 GE 528503
(43) Date of publication of application: 12.07.2006
(73) Proprietor: CAMELYN LTD, Tbilisi 0145 (GE)
(72) Inventor: ABASHIDZE, Guguli, Tbilisi, 0179 (GE); MAGLAKELIDZE, Ketino, Tbilisi, 0179 (GE); KADAGISHVILI, Eliso, Tbilisi, 0179 (GE)
(74) Representative: Benatov, Emil Gabriel
(86) International application number: PCT/GE2004/000004
(87) International publication number: WO 2005/034969

(56) References cited:
- WO-A-00/39135
- WO-A-01/67888
- WO-A-02/30467

## Description

Proposed invention relates to medicine and refers to the medicinal means prepared on the bases of honey, methods for use and obtaining thereof.

Application of honey in medicine has been known for a long time. Honey has immunostimulating, bactericidal, granulation stimulating and regenerative effects. On the basis of the said effects honey is applied successfully for treatment of wounds, trophic ulcers, ulcerous diseases and immunodeficiency. There is also known application of honey for treatment of kidneys and nervous system. In spite of such wide therapeutic effects of honey it is not efficient for treatment of tumor.

There is known a preparation having medicinal effect against tumor and metastasis (RU 2132689 (Tulev Y.) 10.07.99) comprising extract of composite family plant, honey and propolis. It is used for treatment and prevention of tumors developed due to immunodeficiency.

The drawback of the above preparation is that it is less effective for the initial tumor processes.

Application of chemotherapeutic preparations for treatment of tumors is also commonly known (Mashkovskij M.D., Medicinal Means, M., New Wave, v. 2, p.406-431).

Chemotherapeutic preparations are sufficiently toxic, are characterized with many serious complications and in result their application is very limited, whereas, in many far gone cases of tumor they are inefficient. In addition, they do not improve the quality of patient's life.

The technical effect of the invention is in increasing the medicinal effect, widening of the therapeutic spectrum and prevention of complications.

The essence of the invention is in that the honey is treated thermally and the obtained solution is settled. Then the solution is mixed with adsorbent and again settled, further it is filtered. The obtained filtrate represents a soluble medicinal means pH of which is 3-4. To receive a dry powder the soluble filtrate is mixed with a pharmaceutically acceptable auxiliary means and the obtained mixture is dried. The soluble medicinal means and the dry powder are used as a bactericidal, antiviral, immunostimulating, anti-blastoma and regenerative means. An ointment contains the above mentioned soluble medicinal means, propolis, oil of St-John's wort, beeswax and water. The said ointment is used for treatment of wounds of skin and soft tissues, burns, purulent processes and trophic ulcers.

According to the invention the honey is treated at 100-160°C temperature, settled during 22-26 hours, as an adsorbent and activated carbon is used ratio (mass/volume) of which with the liquid is 7:100, whereas, the liquid is preferably heated before mixing with the adsorbent, after mixing it is settled for 10-14 hours. It is preferably filtered twice. In the preferable embodiment of the invention as an auxiliary means is used sodium bicarbonate (NaH₂CO₃) mixed with the soluble medicinal means mass ratio 1:1.

One aspect of the invention is an injection solution representing the above soluble medicinal means. It is obtained as follows: honey is treated thermally at 100-160°C temperature and the obtained solution is settled during 22-26 hours, 24 hours are preferable. After expiration of the above period of time dark yellow solution is received. The said solution is heated, activated carbon added, ration 100:7 and stirred for about five minutes afterwards it is settled for 10-14 hours, 12 hours are preferable. After expiration of the above time period the mixture is filtered. For filtering Pasteur-Chamberland threefold filter is used preferably. Filtration is performed preferably twice. Finally, filtrate of light yellow color is obtained with specific smell, bitter taste and pH 3-4. The injection solution is valid for more than ten years.

One more aspect of the invention is a dry powder obtained from the soluble medicinal means and pills and capsules made on the bases thereof. The dry powder is obtained in the following way: the soluble filtrate obtained in result of the above processes is mixed with the pharmaceutically acceptable auxiliary means. It may be one of the excipients commonly known in pharmaceutical industry, but preferably sodium bicarbonate is used. Mixing of the solution and sodium bicarbonate is carried out in equal quantities. After mixing the mixture is stirred well. Finally, a mush-like thick mass is received that is then dried. Drying may be performed in many ways, but preferable drying on a water-bath is preferable (due to simplicity and cheapness). The dry mass obtained in result of drying is pounded up to receiving a homogeneous fine powder. Finally, brown powder is obtained with bitter taste and alkali reaction. The said powder is placed in gelatin capsules. For receiving pills the pharmaceutically acceptable filling agents are mixed and pelletizing is performed in the pharmaceutical industry under the known methods. As a filling agent may be used talc powder, starch, microcrystalline cellulose, etc. In the preferable embodiment of the invention capsules and pills contain 0,3 or 0,6 g active ingredient. Pills and capsules are kept in the dry dark place, is valid for more than ten years.

One more aspect of the invention is an ointment containing the above soluble medicinal means, propolis, St.-John's wort oil, beeswax and water. In the most preferable embodiment of the invention the ointment contains components in the following ratio of mass portions:

| | |
|---|---|
| Medicinal means | 3-5 |
| Propolis | 2-4 |
| Oil of St.-John's wort | 9-11 |
| Beeswax | 6-8 |
| Water | 4-6 |

The ointment is prepared in the following way: beeswax and propolis are melted on the water-bath, St.-John's wort oil is added and stirred well. Further the mixture is cooled and medicinal means and water are added, distilled water is preferable. The mixture is again stirred until obtaining a homogeneous mass after this the mixture is poured in a container and cooled. Finally, a semi-stable ointment of yellow color and specific smell is obtained, valid for more than ten years.

The above preferable embodiments explain and not limit the scope of protection of the invention. It is readily understandable that proceeding from the proposed invention other alternate embodiments may be made that do not exceed the scope of the invention.

Therapeutic characteristics of the injection solution, pills and capsules are: bacterial and viral infections (due to their bactericidal, antiviral and immunostabilizing effect), wounds (regeneration effect) and tumor processes (antiblastoma effect). Their application is especially actual and effective for treatment of tumor (tumor, sarcoma, etc.) and prevention of relapse.

Injection solution is introduced intravenously or in muscles. 5-10% solution of the medicinal means is introduced in vein, for the purpose before introduction the injection solution is dissolved in the injection solution or physiological solution. Its doze is 1g 2-4 times a day. 35% solution of the medicinal means is introduced in the muscles, for the purpose before introduction the injection solution is dissolved in the injection solution or novocaine solution, novocaine is preferable. Its doze is 1g 2-4 times a day. Duration of the parenteral treatment course is 20-30 days. Rest period between courses must be at least eight days.

Capsules and pills are introduced perorally, doze 0,3-0,6g 3-5 times a day, daily doze is 1,5-2g.

The ointment is applied locally and its therapeutic characteristics are: wounds, burns, purulent processes of skin and soft tissues, trophic ulcers. Presumably, the ointment must be effective for treatment of skin tumors, though; confirmation of the mentioned requires further research. For the purpose of treatment the ointment is applied as follows: the injured place is covered with the cotton fabric impregnated with the ointment and fixed. In the case of deep wounds the ointment is introduced with a tampon. Duration of treatment with the ointment is 7-14 days in average.

The proposed medicinal means was tested experimentally as well as clinically.

### STUDY OF REGENERATION PROPERTIES

Under the aseptic rules dogs were wounded. Part of dogs (research group) was treated with the soluble medicinal means, part (control group) was not treated, further the wound was closed fast. After six, twelve, forty-eight and seventy two hours research, as well as control group, dogs' wounds surface by biopsy material was taken for studying the regeneration process under the microscope. Results of the experiment were the following; during the first hours in the material taken from the research animals development of granulation tissues was marked, that was in different stages of maturing. In the materials taken after 48-72 hours matured granulation tissues was marked, large quantity of fibrocytes and delicate fibrous tissue. Blood elements were marked in very small quantities, as well as with small quantity of blood vessels.

In the material taken from the control group granulation tissue was marked rich with blood elements, especially leucocytes. Elements of connective tissue were less developed.

### STUDY OF BACERICIDAL ACTIVITY

Action of the preparation on 64 different microbe culture (staphylococcus, colon bacillus, microbe typhoparatyphosus, dysentery bacillus) was studied. Bacteria were cultivated on the nutrient medium where the proposed means was mixed in different concentrations (1,25%, 2,5%, 5%, 10%, 20% and 40%).

Following results were obtained: 5% preparation revealed bactericidal activity in 24 hours, 10% - 4 hours, 15% - in an hour, 20% - 30 minutes and 40% - 15 minutes.

### STUDY OF ANTIVIRAL ACTIVITY

Action of the preparation on rabies virus was studied. Testing was performed for 16 hares (8 research and 8 control). To the control group hares laboratory virus was introduced subdural in the dilution 1:20, quantity 0,2ml. To the research group hares the same virus was introduced diluted (ratio 1:20) in the 20% solution of the preparation. Within five days from introduction of the virus all the animals of the control group died revealing full clinic of rabies. From the control group all the animals survived, three of them developed paresis of extremities without any feature of rabies.

In the second series of research four hares were introduced subdural a virus of rabies and after 24 hours from introduction they were treated with intravenous injections of 20% solution of the preparation. On the fifth day two revealed the features of rabies and died, two survived.

### STUDY OF ANTIBLASTOMA ACTIVITIES

Experiment was performed for A line mice, rats and mice with spontaneous tumor of mammary glands, as well as tumors for transplantation 1) mouse sarcoma and 2) rat sarcoma.

Research and control animals equal in view of sex and age were selected, they were kept in absolutely same conditions. In order to exclude a reflex action of the preparation introduction process the control animals were introduced a physiological solution in the same quantity, as the quantity of preparation introduced for the research animals. Preparation as well as the physiological solution was introduced on the side opposite to the tumor. The results were considered according to the terms of revealing the initial features of tumor, size of tumor and histomorphologic research.

In result of treating the spontaneous tumor with the preparation from the third in every case reduction of tumor was marked, during the remaining time (two months) the tumor reduced gradually, and in 25% it disappeared.

At histomorphologic examination necrosis of tumor tissue and mircoscopical hemorrages were marked.

### Effect of the preparation on mice sarcoma

The preparation was introduced after 24 and 28 hours from transplantation of tumor.

After eight days from transplantation of tumor in the control group on the place of transplantation visible tumor nodules were marked, on the tenth day the tumor was large enough.

In the small part of research animals on the ninth day tumor nodules were marked. In this group tumor growth was suppressed during the experiment period.

### Effect of the preparation on rats' sarcoma

The preparation was introduced after 24 hours from tumor transplantation.

In the control group after 48 hours from transplantation of tumor on the place of transplantation tissue induration was marked and tumor nodules were revealed.

In the research group one third of animals displayed tumor nodules on the third day from transplantation and they disappeared on the tenth day of treatment.

### CLINICAL TESTS OF THE PREPARATION ON TUMORS

### Example 1

Patient Kh.S., 43 years old, diagnosis: larynx tumor

Was ill during a year. At receipt in the clinic complained about heavy breathing, cyanosis was marked, pulse - 120. Tracheotomy was conducted immediately, in result breathing was restored.

By histomorphological examination a planocellular epithelial tumor with keratinization was established.

Laryngoscopy: sharp tumefaction in the area of arytenoid cartilage, for this reason cords were not seen.

The patient was treated with the preparation. Within treatment four days of after laryngoscopy reduction of tumefaction was marked and vocal pore opened. The patient was able to breath at closing of tracheotomy tube.

After 38 days from beginning the treatment laryngoscopy was conducted: in the area of arytenoid cartilage and left cord slight tumefaction was marked, at phonation cords move well, tumor - not marked. The tracheotomy tube was removed, the voice was restored, speaks easily. Left the clinic in good health.

### Example 2

Patient B.G., 25 years old, diagnosis: larynx tumor.

Was ill during two weeks. Lost voice, complained about ache at swallowing.

Laryngoscopy: hyperemia and thickening of both vocal cords were marked, on the left vocal cord tumor formation was marked. Biopsy was carried out.

By histomorphological examination a planocellular epithelial tumor with keratinization was established.

The patient was treated with the preparation. Within treatment fourteen days of treatment the pain disappeared, voice - restored.

Laryngoscopy: hyperemia and tumefaction of the cords were not marked, in the place of tumor - an insignificant tumefaction.

After 24 days of treatment the tumor disappeared. Left the clinic in good health. After seven years he feels well.

Clinical checkup was performed for 12 people diseased with larynx tumor, two of them had metastasis in a lung. In result of treatment with the preparation the state of their health improved, in four persons the tumor disappeared, as well as metastasis in the lung of the two persons.

### Example 3

Patient A.A., 48 years old, diagnosis: gullet tumor.

Was ill during three months. Complained about dysphagia, feeling of foreign matte, heavy breathing.

Esophagoscopy: near the first narrowing of esophagus tumefaction and 2-3cm long tumor formation with uneven surface was marked.

By histomorphological examination a planocellular epithelial tumor with keratinization was established.

X-ray examination: the contrast substance passes almost completely in the gullet, at the act of swallowing deficiency of filling was seen and retaining of the contrast for a certain period marked.

The patient was treated with the preparation. During 22 days of treatment dysphagia disappeared.

X-ray examination and esophagoscopy were repeated.

Esophagoscopy: tumor was not marked along the whole length of the gullet.

X-ray examination: the contrast passes absolutely freely in the gullet.

The patient left the clinic in good health. After seven years he feels well.

### Example 4

Patient B.V., 67 years old, diagnosis: tumor of the right lung.

Was ill during a month. Rose in temperature, began coughing with blood-spitting, was treated with antibiotics but without results. In the clinic X-ray examination, rontgenography and tomography were carried out.

Conclusion: on the right between the second and fifth ribs laterally was seen an uneven form sharply enclosed shadow that on the lateral X-ray had an oval form and was located in the middle of the lung. Shadow marked in the tomogram was seen on every section, especially on the 8cm depth, size of the shadow was 5X7cm. Picture displayed the existence of peripheral tumor of the right lung.

The patient was treated with the preparation. The preparation was introduce with injections in the muscles 1g 3-times a day. The patient as well took pills 0,3g 3-times a day. During 15 days of treatment the temperature was normalized, cough reduced, blood-splitting stopped. Within a month of treatment the patient felt healthy.

Repeated rontgenography did not show the tumor.

The patient left the clinic in good health.

### Example 5

Patient G.T., 12 years old, diagnosis: sarcoma of the left hip.

Histomorphological diagnosis: fibrosarcoma.

Was ill during three months. On the lower third of the left hip large size tumor was developed. Tumor was operated. After two month from the operation the tumor relapsed in the same place, with this reason he applied to the clinic.

He complained about pains in the left hip. Objectively on the hind surface of the left hip large size immovable tumor was marked, painful with palpation.

The patient was treated with the preparation. During 25 days of treatment the tumor disappeared.

### Example 6

Patient Ch.K., 60 years old, initial diagnosis: lymphogranulomatosis, aplastic anemia, diabetes mellitus.

Came to clinic in heavy state, was considered to be hopeless.

Was ill during four months. The disease began with the rise in temperature, fever. Were not able to diagnose during a month, then lymphogranulomatosis was suspected and puncture of lymph node was made.

Conclusion: reticulum cell sarcoma, the second diagnosis: lymphogranulomatosis.

The state of patient became heavier and she applied to clinic for X-ray therapy, but the said therapy was not carried out - the blood picture sharply became heavy. Anti-anemia and hormonal treatment had no results.

On the X-ray of the chest on the right to the third rib not uniform shadow of average intensity was marked, and from the seventh rib - homogeneous shadow.

After puncture of marrow aplastic state was established.

After puncture of the lymph nodes of the neck on the background of erythrocytes, giant non-mature cells of lymphoreticular type in large quantities were marked.

The patient complained about common asthenia, insufficiency of air, heavy breathing, worsening of sight, bad appetite, pains in the perineum.

The patient was treated with the preparation, all the prescriptions were terminated. During the seven days of treatment the state of the patient improved, but infiltration on the left shoulder and buttock (caused by earlier injections) suppurated, with this reason infiltration opened and pus in large quantities evacuated. The wounds were washed with 10% solution of the preparation and the tampon impregnated in the solution was left in the wound. After few days the pus disappeared and wounds were healed. In parallel with the above actions treatment of the patient with the preparation continued, in particular, the patient was injected three times a day and the patient took pills 0,5g 3-times a day. After a month from the beginning of treatment the patient was taking only the pills. Duration of the treatment was three months and a half, after the X-ray was repeated that did not reveal a pathologic process. The treatment was ended. The patient left the clinic in good health. After two years she is feeling well.

### Example 7

Patient B.V., diagnosis: gastroptosis.

Histomorphological diagnosis: reticuloblastoma.

The patient complained about the common asthenia, vomiting and pains in epigastrium. With palpation in epigastrium painful tumor formation was marked.

The patient was treated with the preparation. He was injected in muscles 1 g 3-times a day, he also took ferrum preparations. He tool four courses of treatment, the complaints were removed, gained weight, with palpation tumor was not felt. The patient left the clinic.

In a year and a half pains in the epigastrium began, laparatomy was made, on the minor curvature of the stomach 6X6cm tumor was marked. Stomach resection was made. After the operation he was took the preventive course of the preparation. After two years from the operation he feels well.

### Example 8

Patient K.V., 69 years old, diagnosis: urinary bladder tumor.

The patient complained about strangury, urine with blood.

Cystoscopy: inflammatory changes in the urinary bladder, on the hind wall papular tumor were marked. The operation was made. After pathomorphologic examination of the cutout material carcinoma of the urinary bladder was revealed.

Within a month after operation the blood urination began.

Cystoscopy: tumor formation on the hind wall of the urinary bladder.

The patient was treated with the preparation. He was injected in the muscles 1g 3-times a day, he also took pills 0,3g 3-times a day. Within 7 days of treatment hematuria stopped, the patient gained weight. After 40 days of treatment he left the clinic in good health. After eight years he feels well.

The preparation was tested on the six persons diseased with the tumor of the urinary bladder. In all the cases good results were obtained.

### Example 9

Patient G.O., 34 years old, diagnosis: tumor of prostate.

The patient complained about strangury, frequent urination. Was ill for a month.

After examination - the prostate was increased sharply, flexible consistence.

Cystostomy was made and he was treated with the preparation. During 15 days of treatment urination was normalized, the patient gained 10kg of weight. After two months of treatment catheter was removed and fistula sewn. The patient left the clinic in good health, after a year he feels satisfactory.

### Example 10

Patient A.M., 48 years old, diagnosis: tumor of the cervix of the uterus.

Histomorphological diagnosis: adenocarcinoma.

The patient complained against the general asthenia, bloody discharges. After gynecological examination flexible, uneven surface, painful bleeding tumor formation was revealed.

The patient was treated with preparation. She was injected in the muscles 1 g 3-times a day. After 25 days of treatment the state of the patient improved, bloody discharge stopped, size of the tumor formation reduced. After the second course of treatment the patient feels herself healthy, after repeated gynecological examination tumor was not fixed.

In addition to the above examples, the preparation was tested on 59 persons who had tumor processes of different localization and morphology. In all the cases positive results were marked.

On the basis of analysis of experimental and clinical results we may conclude that: 1) the preparation has antibacterial, antiviral, anitblastoma properties and is a strong bio-stimulator for the organism; 2) preparation is not toxic and has no side effects; 3) the preparation displays especially antiblastoma properties in the initial stages of tumor; 4) preparation must be taken perolally for prevention of relapses, especially, in the cases of sarcoma; 5) in the terminal stages of tumor the preparation improves the general state and life quality, prolongs life, in isolated instances recovers completely.

The ointment was tested on 21 persons, five of them had aseptic wounds, six - purulent wounds, three - burns, five - trophic ulcers, two - bedsores. In all the cases the patients recovered completely, without complications, healing was with delicate scars.

Thus, the proposed medicinal preparations represent effective cheap means for treatment of number of serious and heavy pathological processes.

## Claims

1. A method of obtaining a liquid medicinal means is that honey is treated thermally at 100-160 C temperature, the obtained solution settled, then the solution is mixed with an adsorbent and settled again, afterwards filtered.

2. The method of claim 1 is in that the liquid obtained in result of thermal treatment is settled during 22-26 hours.

3. The method of claim 1 is in that before mixing with adsorbent the mixture is heated.

4. The method of claims 1, 3 is in that after mixing with adsorbent the mixture is settled during 10-14 hours.

5. The method of claims 1, 3 and 4 is in that ratio of adsorbent and the liquid is 7: 100.

6. The method of claims 1, 3 and 5 is in that the adsorbent is an activated carbon.

7. The method of claim 1 is in that the filtering is performed twice.

8. A medicinal means **characterized in that** it is obtained under the method of any claims 1 - 7 and its pH is 3-4.

9. The medicinal means of claim 8 **characterized in that** it is an injection solution.

10. The medicinal means of claim 8 **characterized in that** it is used for biostimulation, for treatment and prevention of microbial and viral infections, tumor processes.

11. The medicinal means according the claim 10 **characterized in that** it is used for prevention of tumor processes relapse.

12. A method for obtaining a dry powder is in that the medicinal means of claim 8 is mixed with the pharmaceutical acceptable auxiliary means, the obtained mixture is dried and the dry mass is pounded.

13. The method according the claim 12 is in that the mass ratio of the medicinal means and the pharmaceutically acceptable auxiliary means is 1: 1.

14. The method according the claims 12-13 is in that pharmaceutical acceptable auxiliary means is sodium bicarbonate.

15. A medicament containing an active ingredient placed in a capsule is **characterized in that** as an active ingredient contains the powder obtained under the method of claims 12-14.

16. The medicament according the claim 15 is **characterized in that** it contains the active ingredient in the quantity of 0,3-0,6g.

17. The medicament according the claims 15 and 16 is used for biostimulation, for treatment and prevention of microbial and viral infections and tumor processes.

18. The medicament according the claim 17 is used for prevention of tumor processes relapse.

19. A pill containing the active ingredient and the pharmaceutical acceptable filling agent **characterized in that** as an active ingredient contains the powder obtained under the method of claims 12-14.

20. The pill according the claim 19 is **characterized in that** it contains the active ingredient in the quantity of 0, 3-0,6g.

21. The pill according the claims 19 and 20 is used for biostimulation, for treatment and prevention of microbial and viral infections and tumor processes.

22. The pill according the claim 21 is used for prevention of the tumor processes relapse.

23. An ointment is **characterized in that** it contains the medicinal means according the claim 8 propolis, St. -John's wort oil, beeswax and water.

24. The ointment according the claim 23 is **characterized in that** it contains components in the following ratio of mass portions:
| | |
|---|---|
| Medicinal means | 3-5 |
| Propolis | 2-4 |
| Oil of St. -John's wort | 9-11 |
| Beeswax | 6-8 |
| Water | 4-6 |

25. The ointment according the claims 23 and 24 is used for treatment of wounds of skin and soft tissues, burns, purulent processes and trophic ulcers.

## Patentansprüche

1. Verfahren, das die Herstellung eines flüssigen medizinischen Mittels erlaubt, **dadurch gekennzeichnet, dass** Honig einer thermischen Behandlung mit Temperaturen zwischen 100 - 160 °C unterzogen wird, die erhaltene Lösung sich absetzen gelassen wird, wonach die Lösung mit einem Absorptionsmittel gemischt und sich noch einmal absetzen gelassen wird, wobei eine Filtration am Ende verwirklicht wird.

2. Das Verfahren nach Anspruch 1, **dadurch gekennzeichnet, dass** die durch die thermische Behandlung erhaltene Flüssigkeit sich während 22 - 26 Stunden abgesetzt wird.

3. Das Verfahren nach Anspruch 1, **dadurch gekennzeichnet, dass** die Mischung vor dem Mischen mit dem Absorptionsmittel erwärmt wird.

4. Das Verfahren nach Ansprüchen 1 und 3, **dadurch gekennzeichnet, dass** die Mischung sich nach dem Mischen mit dem Absorptionsmittel während 10 - 14 Stunden abgesetzt wird.

5. Das Verfahren nach Ansprüchen 1, 3 und 4, **dadurch gekennzeichnet, dass** die Proportion zwischen dem Absorptionsmittel und der Flüssigkeit gleich 7 : 100 ist.

6. Das Verfahren nach Ansprüchen 1, 3 und 5, **dadurch gekennzeichnet, dass** das Absorptionsmittel Aktivekohle ist.

7. Das Verfahren nach Anspruch 1, **dadurch gekennzeichnet, dass** die Filtration zweimal verwirklicht wird.

8. Medizinisches Mittel, **dadurch gekennzeichnet, dass** es durch das Verfahren nach einigen der Ansprüche 1 - 7 erhalten wird und dass sein pH gleich 3 - 4 ist.

9. Das medizinische Mittel nach Anspruch 8, **dadurch gekennzeichnet, dass** es eine zum Einspritzen bestimmte Lösung ist.

10. Das medizinische Mittel nach Anspruch 8, **dadurch gekennzeichnet, dass** es zur Biostimulation, zur Behandlung und Vorbeugung von Mikroben- und Vireninfektionen, von tumoralen Vorgängen.

11. Das medizinische Mittel nach Anspruch 10, **dadurch gekennzeichnet, dass** es zur Vorbeugung des Rezidivs von tumoralen Vorgängen verwendet wird.

12. Verfahren, das die Herstellung eines trockenen Pulvers erlaubt, **dadurch gekennzeichnet, dass** das medizinische Mittel nach Anspruch 8 mit dem pharmazeutisch akzeptabelen Hilfsmittel gemischt, die erhaltene Mischung getrocknet und die trockene Masse zerreibt wird.

13. Das Verfahren nach Anspruch 12, **dadurch gekennzeichnet, dass** die Proportion zwischen der Masse des medizinischen Mittels und dieser des pharmazeutisch akzeptabelen Hilfsmittels gleich 1 : 1 ist.

14. Das Verfahren nach Ansprüchen 12 - 13, **dadurch gekennzeichnet, dass** das pharmazeutisch akzeptabele Hilfsmittel Natriumhydrogenkarbonat ist.

15. Medikament, das in eine Kapsel eingesetzte aktive Komponente enthält, **dadurch gekennzeichnet, dass** es als aktive Komponente das durch das Verfahren nach Ansprüchen 12 - 14 erhaltene Pulver enthält.

16. Das Medikament nach Anspruch 15, **dadurch gekennzeichnet, dass** es die aktive Komponente in einer Menge von 0,3 - 0,6 g enthält.

17. Das Medikament nach Ansprüchen 15 und 16, **dadurch gekennzeichnet, dass** es zur Biostimulation, zur Behandlung und Vorbeugung von Mikroben- und Vireninfektionen und tumoralen Vorgängen verwendet wird.

18. Das Medikament nach Ansprüchen 15 und 16, **dadurch gekennzeichnet, dass** es zur Vorbeugung des Rezidivs von tumoralen Vorgängen verwendet wird.

19. Pille, die die aktive Komponente und den pharmazeutisch akzeptabelen Füllstoff enthält, **dadurch gekennzeichnet, dass** sie als aktive Komponente das durch das Verfahren nach Ansprüchen 12 - 14 erhaltene Pulver enthält.

20. Die Pille nach Anspruch 19, **dadurch gekennzeichnet, dass** sie die aktive Komponente in einer Menge von 0,3 - 0,6 g enthält.

21. Die Pille nach Ansprüchen 19 und 20, **dadurch gekennzeichnet, dass** sie zur Biostimulation, zur Behandlung und Vorbeugung von Mikroben- und Vireninfektionen und tumoralen Vorgängen verwendet wird.

22. Die Pille nach Anspruch 21, **dadurch gekennzeichnet, dass** sie zur Vorbeugung des Rezidivs von tumoralen Vorgängen verwendet wird.

23. Salbe, **dadurch gekennzeichnet, dass** sie das medizinische Mittel nach Anspruch 8, Propolis, Öl von echter Johanniskraut, Bienenwachs und Wasser enthält.

24. Die Salbe nach Anspruch 23, **dadurch gekennzeichnet, dass** sie Komponenten in der folgenden Proportion der Masseanteile enthält:
| | |
|---|---|
| Medizinisches Mittel | 3 - 5 |
| Propolis | 2 - 4 |
| Öl von echter Johanniskraut | 9 - 11 |
| Bienenwachs | 6 - 8 |
| Wasser | 4 - 6 |

25. Die Salbe nach Ansprüchen 23 und 24, **dadurch gekennzeichnet, dass** sie zur Behandlung von Haut- und Weichgewebenwunden, Verbrennungen, purulenten Vorgängen und trophischen Geschwüren verwendet wird.

## Revendications

1. Méthode permettant d'obtenir un moyen médicinal liquide, **caractérisée en ce que** du miel est soumis à un traitement thermique par une température de 100 - 160 °C, la solution obtenue est laissée se précipiter, après quoi ladite solution est mélangée avec un absorbant et laissée se précipiter de nouveau, en la filtrant à la fin.

2. La méthode selon la revendication 1, **caractérisée en ce que** le liquide obtenu par suite du traitement thermique se précipite pendant 22 - 26 heures.

3. La méthode selon la revendication 1, **caractérisée en ce que** la mixture est chauffée avant d'être mélangée avec l'absorbant.

4. La méthode selon les revendications 1 et 3, **caractérisée en ce qu'**après le mélangeage avec l'absorbant la mixture se précipite pendant 10 - 14 heures.

5. La méthode selon les revendications 1, 3 et 4, **caractérisée en ce que** la proportion entre l'absorbant et le liquide est égale à 7 : 100.

6. La méthode selon les revendications 1, 3 et 5, **caractérisée en ce que** l'absorbant est charbon actif.

7. La méthode selon la revendication 1, **caractérisée en ce que** la filtration s'accomplit deux fois.

8. Moyen médicinal, **caractérisé en ce qu'**il est obtenu par la méthode selon quelconques des revendications 1 - 7 et **que** son pH est égal à 3 - 4.

9. Le moyen médicinal selon la revendication 8, **caractérisé en ce qu'**il représente une solution d'injection.

10. Le moyen médicinal selon la revendication 8, **caractérisé en ce qu'**il est utilisé pour la biostimulation, pour le traitement et la prévention des infections microbiales et virales, des processus tumorals.

11. Le moyen médicinal selon la revendication 10, **caractérisé en ce qu'**il est utilisé pour la prévention de la rechute des processus tumorals.

12. Méthode permettant d'obtenir une poudre sèche, **caractérisée en ce que** le moyen médicinal selon la revendication 8 est mélangé avec le moyen auxiliaire qui est pharmaceuticalement acceptable, la mixture obtenue est desséchée et la masse sèche est moulue.

13. La méthode selon la revendication 12, **caractérisée en ce que** la proportion entre la masse du moyen médicinal et celle du moyen auxiliaire qui est pharmaceuticalement acceptable est égale à 1 : 1.

14. La méthode selon les revendications 12 - 13, **caractérisée en ce que** le moyen auxiliaire qui est pharmaceuticalement acceptable est bicarbonate de soude.

15. Médicament contenant un ingrédient actif qui est placé dans une capsule, **caractérisé en ce qu'**il contient comme ingrédient actif la poudre obtenue par la méthode selon les revendications 12 - 14.

16. Le médicament selon la revendication 15, **caractérisé en ce qu'**il contient l'ingrédient actif en une quantité de 0,3 - 0,6 g.

17. Le médicament selon les revendications 15 et 16, **caractérisé en ce qu'**il est utilisé pour la biostimulation, pour le traitement et la prévention des infections microbiales et virales et des processus tumorals.

18. Le médicament selon les revendications 15 et 16, **caractérisé en ce qu'**il est utilisé pour la prévention de la rechute des processus tumorals.

19. Pilule contenant l'ingrédient actif et l'agent de remplissage qui est pharmaceuticalement acceptable, **caractérisée en ce qu'**elle contient comme ingrédient actif la poudre obtenue par la méthode selon les revendications 12 - 14.

20. La pilule selon la revendication 19, **caractérisée en ce qu'**elle contient l'ingrédient actif en une quantité de 0,3 - 0,6 g.

21. La pilule selon les revendications 19 et 20, **caractérisée en ce qu'**elle est utilisée pour la biostimulation, pour le traitement et la prévention des infections microbiales et virales et des processus tumorals.

22. La pilule selon la revendication 21, **caractérisée en ce qu'**elle est utilisée pour la prévention de la rechute des processus tumorals.

23. Onguent, **caractérisé en ce qu'**il contient le moyen médicinal selon la revendication 8, du propolis, de l'huile de millepertius perforé, de la cire d'abeilles et de l'eau.

24. L'onguent selon la revendication 23, **caractérisé en ce qu'**il contient des composants dans la proportion des portions de masse suivante :
| | |
|---|---|
| Moyen médicinal | 3 - 5 |
| Propolis | 2 - 4 |
| Huile de millepertius perforé | 9 - 11 |
| Cire d'abeilles | 6 - 8 |
| Eau | 4-6 |

25. L'onguent selon les revendications 23 et 24, **caractérisé en ce qu'**il est utilisée pour le traitement des blessures de la peau et des tissus mous, des brûlures, des processus purulents et des ulcères trophiques.
